# EUROPEAN PATENT APPLICATION

(11) **EP 1 726 286 A1**
(43) Date of publication of application: **29.11.2006**
(21) Application number: 05721415.7
(22) Date of filing: 16.03.2005
(51) Int. Cl.: A61J 1/14, A61M 5/152

(54) **PRESSING BAG AND CONSTANT-PRESSURE PRESSING BAG SYSTEM**

(30) Priority: 16.03.2004 JP 2004116194
(71) Applicant: Kabushiki Kaisha Cycos, Machida-shi, Tokyo 194-0032 (JP)
(72) Inventor: SATO, Chikashi, Machida-shi, Tokyo 194-0032 (JP)
(74) Representative: Goddar, Heinz J.
(86) International application number: PCT/JP2005/005438
(87) International publication number: WO 2005/087174

(57) **Abstract**

A pressure bag (1) includes: a pressing chamber (2) adapted to be inflated by air-feeding; and a rectangular outer layer (3) having such length as to surround the pressing chamber (2). Fasteners (35, 36) are formed on one end side of a front surface and the other end side of a back surface of shorter sides of the outer layer (3). The pressing chamber (2) is fixed to the outer layer (3) in the axial direction. In use, infusion bags (40, 41) are surrounded by the outer layer (3) in a manner contacting with the pressing chamber (2) and fastened by the fasteners (35, 36).

## Description

### Technical Field

The present invention relates to a pressing bag for pressing an infusion bag or a blood-transfusion bag to inject a fluid in such bag into a body of a patient and relates to a constant-pressure pressing bag system for maintaining constant pressing.

### Background Art

A pressing bag is so arranged that an outer layer formed from a sheet or a mesh that is difficult to stretch is fixed on both ends of a pressing chamber formed air-tightly from a sheet that is difficult to stretch, and an infusion bag is set in a pocket formed between the pressing chamber and an outer layer belt, the pressing chamber being inflated by feeding the air to press the infusion bag. The air feeding to the pressing chamber is manually performed using a rubber-bulb pump. In such arrangement, an infusion set is connected to an outlet of the infusion bag, and an infusion rate is typically controlled by adjusting opening/closing of a tube using a clamp or by embedding a narrow tube etc. in a part of an infusion tube circuit as a resistance.

### Disclosure of the Invention

### Problems to be Solved by the Invention

Several sizes of pressing bags are currently being used for infusion bags of 500 ml to 3000 ml. The volume of a pressing chamber used for the 500 ml infusion bag, which is the smallest type, is about 2 litters, which is four times as large as the volume of the 500 ml infusion bag and wastefully bulky. For example, when a pressing bag for the 500 ml infusion bag is used for a 200 ml infusion bag, the volume of pressing chamber will be ten times as large as the volume of the infusion bag. Thus, the currently-used pressing bags are not capable of adjusting the size of the pressing chamber so as to be suitable for an infusion bag to be used.

The currently-used pressing bag is so arranged to allow one infusion bag to be set on one side of the pressing chamber. If two infusion bags can be set on each side of the pressing chamber and used, the number of pressing chambers to be used can be saved, thereby also saving a work for controlling the pressing chambers.

A plastic-bottle type infusion bag is formed in a bottle-like shape. Due to the bottle-like shape and the elasticity of the plastic, the plastic-bottle type infusion bag cannot be pressed sufficiently by the conventional pressing bag of one-side pressing type, which might cause a discharge pressure from the plastic bottle to be lowered. Accordingly, there is required a pressing bag capable of pressing the plastic-bottle type infusion bag without lowering its discharge pressure.

Meanwhile, as for a bag-like pressing chamber which presses an infusion bag from both sides, it is difficult to visually check a remaining amount of the infusion bag. Infusion can be performed at ease if the inner side of the infusion bag can be visually checked in some way.

When the pressing bag is suspended for use, there are problems in which: the infusion bag sandwiched by the outer layer and the pressing chamber might fall down before the pressing chamber is inflated to press the infusion bag against the outer layer; and a position of the infusion bag might be displaced.

For an infusion bag having a large volume and divided into a plurality of sub-bags of which contents are mixed before use, an infusion pump having a large size (both in horizontally and vertically) and a large weight is used. If the pressing bag can be applied to such infusion bag and the size of the pressing bag can be reduced, it becomes convenient for use even during transportation.

The currently-used pressing bag is so arranged as to reduce pressing force of the pressing chamber as the infusion proceeds and the amount of medicinal solution in the infusion bag is reduced. Accordingly, a gauge or the like to show the internal pressure of the pressing chamber is provided to the pressing bag. A health personnel monitors the internal pressure of the pressing chamber with the gauge, and when the internal pressure is lowered, he/she needs to feed the air using the manual rubber-bulb pump in order to maintain pressing force of the pressing bag. Therefore, a work load of the health personnel becomes large.

### Means for Solving the Problem

An object of the present invention is to provide a pressing bag capable of adjusting a volume of a pressing chamber of the pressing bag to be minimum requisite, using a plurality of infusion bags simultaneously and being applicable also to a plastic-bottle type infusion bag, as well as providing a constant-pressure pressing bag system for constantly maintaining pressing force. Hereinafter, a right-left direction will be referred to as a lateral direction, while an up-down direction will be referred to as an axial direction.

Since the pressing chamber and the infusion bag are entirely surrounded by an outer layer, the outer diameter of the pressing bag is restricted, so that the inflation of the pressing chamber is restricted in a space surrounded by the outer layer. The pressing bag of the present invention is so arranged as to be capable of pressing the infusion bag with the minimum requisite inflation volume of the pressing chamber, thus realizing a non-bulky and gas-saving pressing bag.

An outer layer having fasteners for fastening one end side of a front surface and the other end side of a back surface is formed, while a pressing chamber adapted to be inflated by feeding the air is formed. A pressing bag is formed by fixing a fixing portion provided on one end of the pressing chamber to the outer layer. When one infusion bag is so set that its surface is in a full-surface contact with one side of the pressing chamber and the infusion bag and the pressing chamber is entirely surrounded by the outer layer, the outer diameter is restricted, thereby providing the pressing bag capable of pressing the infusion bag with the minimum requisite inflation of the pressing chamber. By setting an infusion bag on each side of the pressing chamber, a pressing bag for pressing two infusion bags simultaneously can be provided.

An outer layer is formed to have the fasteners for fastening one end side of a front surface and the other end side of a back surface of the outer layer, while a pressing chamber adapted to be inflated by feeding the air is formed with a partition fixing portion and partition-periphery welded portion provided at a position substantially dividing in halves the pressing chamber in a lateral direction, the partition-fixing portion and partition-periphery welded portion being each formed in a vertical direction slightly shortly so as not to reach a periphery-welded portion. The outer layer is formed to have such length as to surround the pressing chamber. By fixing the partition fixing portion of the pressing chamber to the outer layer, the pressing chamber having two chambers can be provided. By setting, each in a full-surface contact manner, one infusion bag on each of outer sides of the two chambers and one infusion bag on an inner side defined by the two pressing chambers and by surrounding the chambers and the infusion bags with the outer layer, an outer-layer type pressing bag capable of simultaneously pressing three infusion bags can be provided.

An outer layer is formed to have the fasteners for fastening the one end side of a front surface and the other end side of a back surface of the outer layer, while a pressing chamber adapted to be inflated by feeding the air is formed with a partition fixing portion and partition-periphery welded portion provided at a position substantially dividing in halves the pressing chamber in a lateral direction, the partition fixing portion and partition-periphery welded portion being each formed in a vertical direction slightly shortly so as not to reach a periphery-welded portion, whereby two chambers on the right and left sides of the partition are formed. Bag fixing portions are fixed to the partition fixing portion so that the two chambers on the right and left sides are each folded in the axial direction to be a bag-like shape, thereby forming two bag-like pressing chambers. The outer layer is formed to have such length as to surround the bag-like pressing chambers. By fixing the partition fixing portion of the bag-like pressing chambers to the outer layer, the outer-layer type pressing bag can be provided. When the air is supplied to the bag-like pressing chambers under a condition where a plastic-bottle type infusion bag is set in each of the bag-like pressing chambers and the bag-like pressing chambers are surrounded by the outer layer to restrict the outer diameter, the entire surfaces of the plastic-bottle type infusion bags are pressed. With the arrangement, since the plastic-bottle type infusion bags are pressed more effectively as compared to the one-side pressing type, decrease of a discharge pressure of the bottle becomes more gradual. By setting one infusion bag in each of the two bag-like pressing chambers and one infusion bag between two bag-like pressing chambers and by surrounding the bag-like pressing chambers with the outer layer, another outer-layer type pressing bag capable of simultaneously pressing three infusion bags can be provided.

The pressing bag may also be arranged so that one bag-like pressing chamber is fixed to the outer layer. With the arrangement, by setting one infusion bag in the bag-like pressing chamber and one infusion bag on each of outer surfaces of the bag-like pressing chamber and surrounding the bag-like pressing chamber and the infusion bags with the outer layer, a still-another outer-layer type pressing bag capable of pressing three infusion bags simultaneously can be provided.

A hole for visually checking the inside of the bag-like pressing chamber is formed and a hole peripheral portion is welded to provide an observation window. With the arrangement, the infusion bag in the bag-like pressing chamber can be seen, so that a remaining amount of the infusion bag can be visually checked.

In order to prevent the infusion bag set in the pressing bag from falling down, a fall-top is provided so as to catch a shoulder portion on an outlet side of the infusion bag. The fall-stop may be formed by welding or sewing so that portions in the outer layer and the pressing chamber or bag-like pressing chamber which are in contact with the shoulder portion on the outlet side of the infusion bag are adhered to each other.

For a large-volume infusion bag, a hole through which a neck portion of the infusion bag is inserted to be brought out of the pressing bag is provided to the outer layer. With the arrangement, the pressing bag is formed by folding the infusion bag in a manner holding the pressing chamber on the inner side thereof and surrounding the infusion bag with the outer layer. Since the infusion bag is folded, compact shape and structure can be realized.

By feeding constantly-controlled pressurized gas from a gas feeder to the pressing chamber of these pressing bags of the present invention instead of feeding the air using a manual rubber-bulb pump, a constant-pressure pressing bag system capable of saving gas and labor can be provided.

### Effect of the Invention

The pressing bag is formed by surrounding the pressing chamber and the infusion bag with the rectangular outer layer. When the pressing chamber is inflated by feeding the air, the pressing chamber is restricted in a space surrounded by the outer layer, so that the pressing chamber that can be bulky can be arranged so as to perform pressing with the minimum requisite inflation volume. In addition, by feeding the constantly-controlled pressurized gas from the gas feeder to the pressing chamber of the pressing bag of the present invention, the constant-pressure pressing bag system capable of saving the gas can be provided.

### Best mode for Carrying out the Invention

A preferred embodiment of the present invention will be described below with reference to the attached drawings. Note that pressing chambers and outer layers are formed by common methods unless otherwise described, and sizes of pressing bags vary depending on sizes of infusion bags to be used.

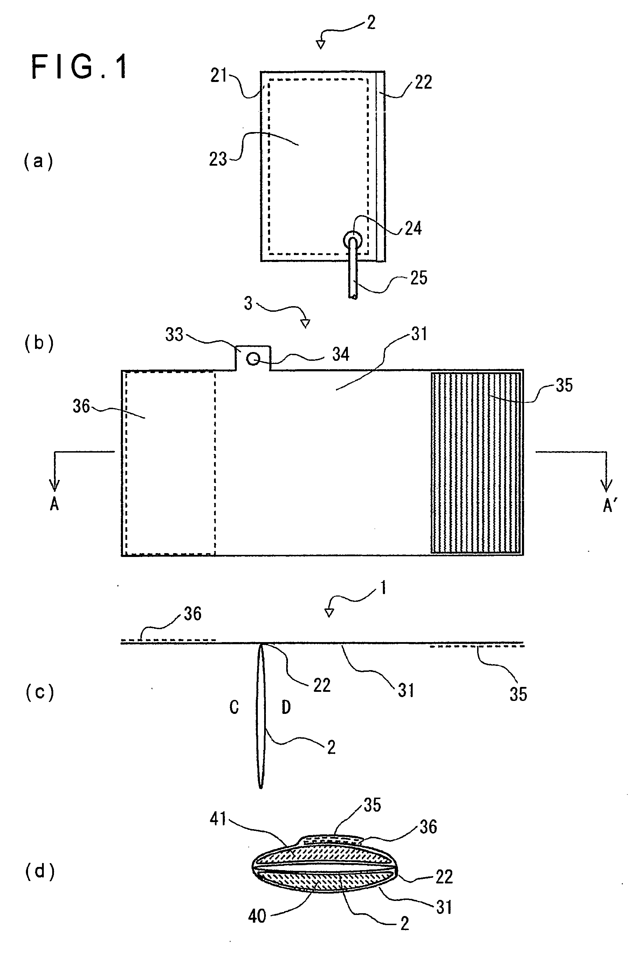

Fig. 1 shows a first embodiment of a pressing bag having one pressing chamber according to the present invention. (a) is a front view of a pressing chamber 2. (b) is a front view of an outer layer 3. (c) is a cross section of a pressing bag 1 that is seen from the line A-A' of the outer layer 3, where a fixing portion 22 of the pressing chamber 2 is fixed to the outer layer 3. (d) is a cross section seen from the line A-A' of the outer layer 3, where infusion bags 40, 41 are set on both sides C, D of the pressing chamber 2 of the pressing bag 1 and surrounded by the outer layer 3. With the outer layer 3 being fastened by planar fasteners 35, 36 provided to both ends of an outer layer belt 31, the pressing chamber 2 is inflated by feeding the air from an air-feeding tube 25.

The pressing bag 1 shown in this figure is formed by fixing in the axial direction the fixing portion 22 of the pressing chamber 2 to the outer layer 3 that has a longer side extending in the lateral direction.

The periphery of the pressing chamber 2 is welded air-tightly to define a periphery-welded portion 21, and the fixing portion 22 is formed on a side in the lateral direction of the periphery-welded portion 21. An inlet 24 for introducing and discharging a pressurized gas is provided air-tightly to a chamber 23, the inlet 24 being connected to the air-feeding tube 25.

The outer layer belt 31 is provided with a suspending portion 33 used for suspending the pressing bag, and a hole 34 is formed in the suspending portion 33. A string-like or strap-like suspending belt may be employed instead of the suspending portion 33. The outer layer belt 31 is provided with the planar fastener 35, 36 on one end side of a front surface and the other end side of a back surface in the lateral direction, the planar fastener 35, 36 being fastened to each other. The outer layer 3 is formed to have a length longer than the width of the pressing chamber 2, the length being long enough to surround the pressing chamber 2 and the infusion bag.

Although the pressing chamber 2 may be made of any material as long as it is air-tight and inflatable, an unstrecheable material is preferable so that the pressing chamber 2 can press the infusion bags 40, 41 when being inflated. The pressing chamber 2 is typically fed with the air and inflated to be 300 mmHg (approx. 40 kPa) in use. Examples of sheet materials that are excellent in pressure resistance and air-tightness may include: a nylon woven fabric coated with a polyurethane or a vinyl chloride; and a sheet obtained by sandwiching a mesh formed from polyethylene yarns by polyvinyl chloride sheets. However, the sheet material is not limited to those described above, but includes any sheet that has excellent pressure resistance and air-tightness. The thickness is preferably around 0.2 to 0.6 mm. The pressing chamber 2 can be provided by overlapping two sheets or folding one sheet and welding the periphery to define the periphery-welded portion 21 to form the chamber 23 into a bag-like shape. Fig. 1 exemplifies an arrangement in which two sheets are overlapped, and therefore the entire periphery is welded air-tightly.

The inlet 24 is preferably a tube with one end having a disc-like shape like an air inlet of an inner tube, but the inlet 24 is not limited thereto. An air-feeding tube 25 is connected air-tightly to the inlet 24. The air-feeding tube 25 itself may be a rubber tube or a plastic tube as long as it has flexibility, air-tightness and pressure resistance.

The outer layer belt 31 is not particularly limited as long as it has flexibility and high tensile strength and is unstrecheable, but the outer layer belt 31 has preferably transparency so that content amounts of the infusion bags 40, 41 can be visually checked. Examples of preferred materials of the outer layer belt 31 may include: a net made of a nylon, a polyester or a polypropylene; and a sheet obtained by sandwiching a mesh formed from polyethylene yarns by vinyl chloride sheets or polyurethane sheets. The thickness of the outer layer belt 31 is preferably around 0.2 to 0.6 mm. The outer layer belt 31 is provided with the planar fasteners 35, 36 on the one end side of the front surface and the other end side of the back surface. The outer layer 3 is formed to have a length that allows the planar fasteners 35, 36 to fasten to each other when the outer layer 3 surrounds the pressing chamber 2 with the infusion bags 40, 41 being set on the both sides C, D of the pressing chamber 2. The fastening may be provided by any fastening unit without limiting to the planar faster.

The fixing portion 22 of the pressing chamber 2 is fixed to the outer layer 3 in Fig. 1. Here, a position for fixing the fixing portion 22 to the outer layer 3 might vary depending on the shape and size of the infusion bag. The fixing may be provided by welding, sewing or bonding.

As shown in Fig. 1(d), the infusion bags 40, 41 are set on the both sides C, D of the pressing chamber 2 and the outer layer 3 entirely surrounds the infusion bags 40, 41 and the pressing chamber 2 and fastens by the planar fastener 35, 36. The outer diameter of the pressing bag 1 is restricted by the surrounding outer layer 3, and the pressing chamber 2 is restricted in a space surrounded by the outer layer 3. While the pressing bag 1 of the present invention has a typical function as a pressing bag for pressing the infusion bags 40, 41, it can perform pressing with a small inflation volume of the pressing chamber 2, thereby realizing the pressing bag 1 that is non-bulky and capable of saving pressurized gas.

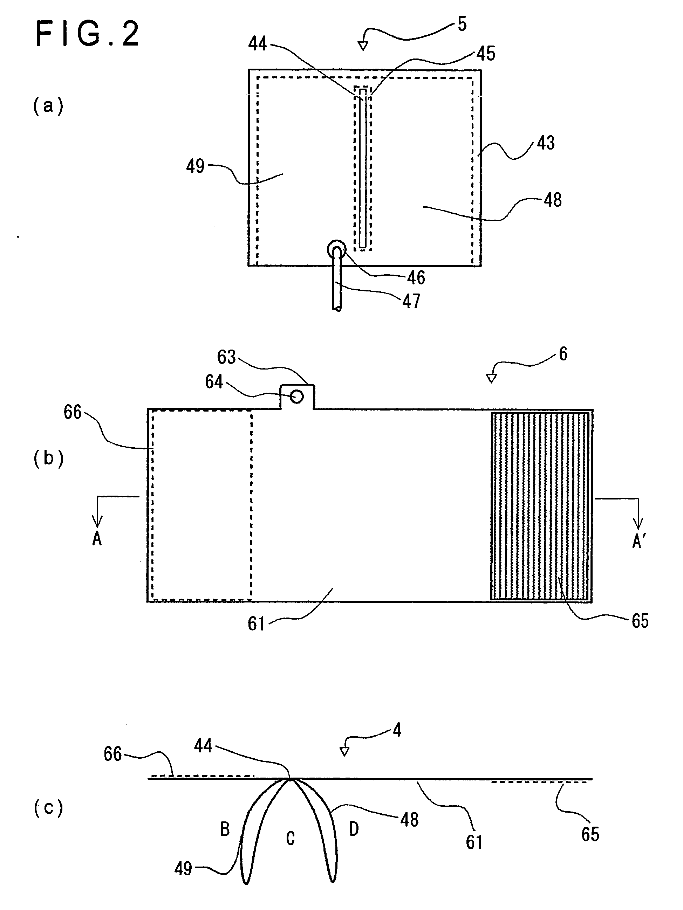

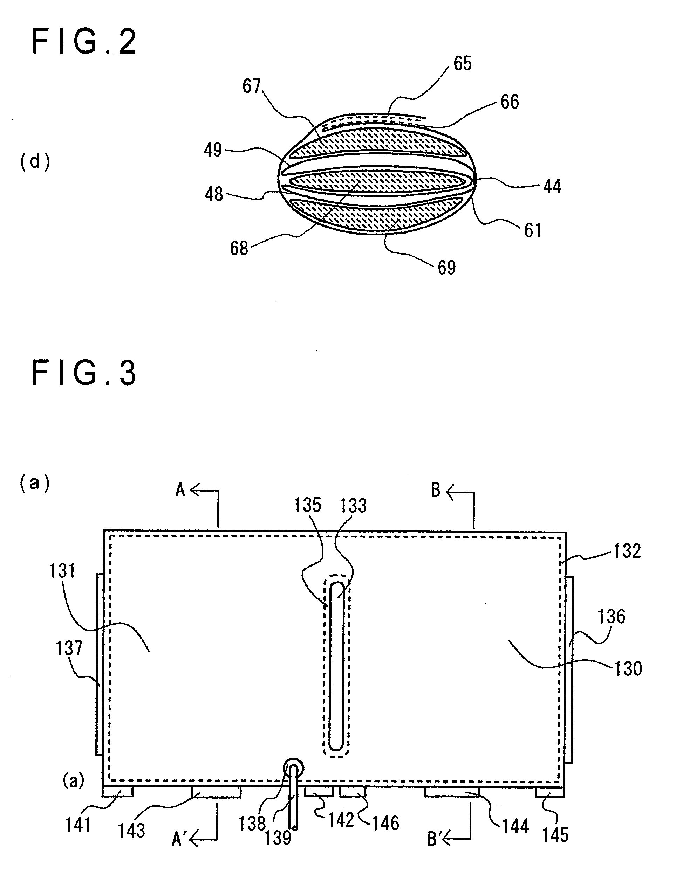

Fig. 2 shows a second embodiment of a pressing bag having a pressing chamber with two chambers. (a) is a front view of a pressing chamber 5, in which one sheet is folded in the axial direction and the periphery thereof is welded air-tightly to define a periphery-welded portion 43, and the pressing chamber 5 is divided into chambers 48, 49 by a partition including a partition-periphery welded portion 44 and a partition fixing portion 45 at a position dividing in halves the pressing chamber 5 in the lateral direction, the partition-periphery portion and the partition fixing portion each being formed slightly shortly so as not to reach the periphery-welded portion 43, the chambers 48, 49 intercommunicating with each other. (b) is a front view of an outer layer 6. (c) is a horizontal cross section of a pressing bag 4 which is seen from the line A-A' of the outer layer 6, in which the partition fixing portion 44 of the pressing chamber 5 is fixed to the outer layer 6. (d) is a horizontal cross section of the pressing bag 4, in which the pressing chamber 5 is surrounded by the outer layer 6 and fastened by the planar fastener 65, 66 after infusion bags 67, 68, 69 are set in B, C, D of the pressing chamber 5, and then the pressing chamber 5 is inflated by feeding the air.

The pressing chamber 5 is arranged so that: one weldable sheet is folded in the axial direction and right, left and upper portions of the periphery are welded air-tightly to define the periphery-welded portion 43, and the pressing chamber 5 is divided into chambers 48, 49 by the partition-periphery welded portion 45 and the partition fixing portion 44 that are each formed at the middle portion slightly shortly so as not to reach the periphery-welded portion 43, the chambers 48, 49 intercommunicating with each other. An inlet 46 is air-tightly formed on the chamber 49, the inlet 46 being connected to a tube 47.

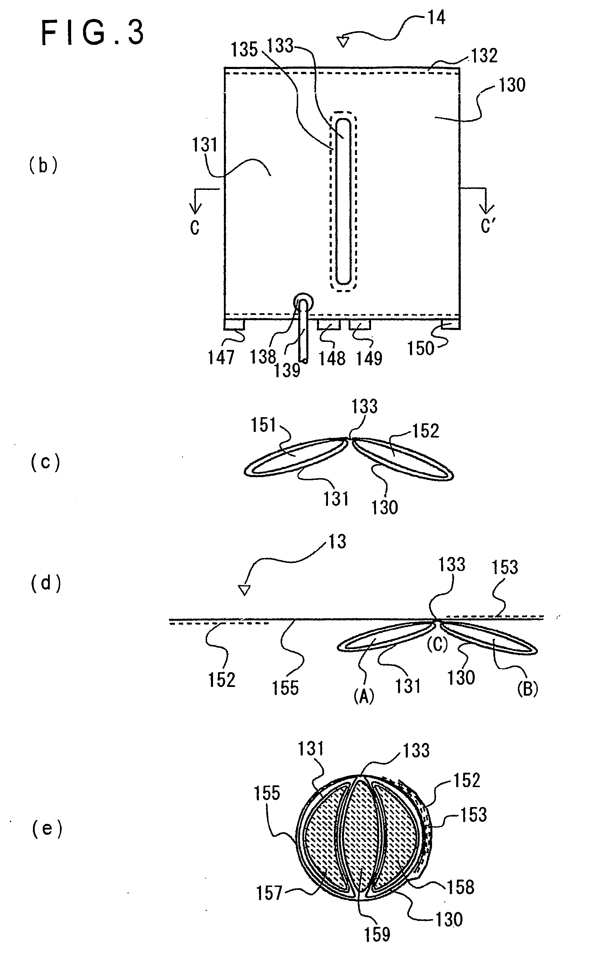

Fig. 3 shows a third embodiment of a pressing bag of the present invention, in which a bag-like pressing chamber 13 having two bag-like chambers is formed. (a) shows the bag-like pressing chamber 14 with the periphery welded air-tightly to define a periphery-welded portion 132, the bag-like pressing chamber being divided into chambers 130, 131 by a partition-periphery portion 133 and a partition fixing portion 135 that are each formed slightly shortly so as not to reach the periphery-welded portion 132 at a position dividing in halves the bag-like pressing chamber in the lateral direction, the chambers 130, 131 intercommunicating with each other. The chamber 130 has a bag fixing portion 136 at an end, and the chamber 131 has a bag fixing portion 137 at an end. The chamber 131 is provided with an inlet 138 to which an air-feeding tube 139 is connected. Stopper belts 141, 142, 143, 144, 145, 146 for preventing infusion bags from falling down are provided on a lower end of the chamber.

(b) is a front view showing the bag-like pressing chamber 13 formed by folding the bag-like pressing chamber 14 toward the back side at the lines A-A' and B-B' in (a), where the bag fixing portions 136, 137 are fixed to the fixing portion 133 to form the chambers 130, 131 in bag-like shapes, the stopper belts 141, 142, 143, 144, 145, 146 being adhered to form fall-stops 147, 148, 149 150. (c) is a horizontal cross section seen from the line C-C' of the bag-like pressing chamber 13 in (b).

(d) shows a pressing bag 13 in which the bag-like pressing chamber 14 is fixed to an outer layer belt 155. (e) is a horizontal cross section, in which infusion bags 157, 158 are set in A, B of the bag-like pressing chambers 14 and an infusion bag 159 is set in C in a sandwiched manner, which are entirely surrounded by the outer layer belt 155 and fastened by planar fasteners 152, 153.

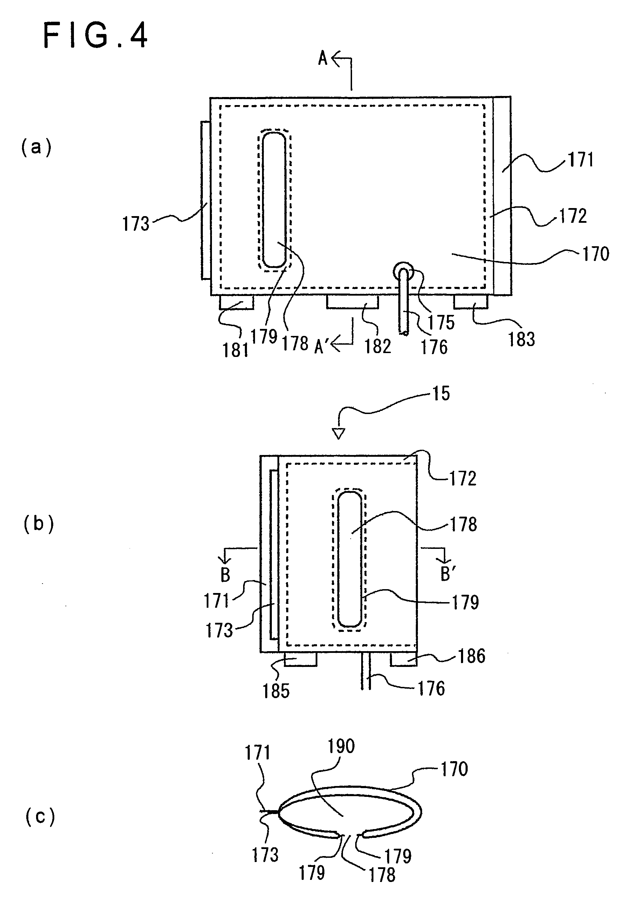

Fig. 4 shows a bag-like pressing chamber 15 provided with an observation window 178. (a) shows a chamber 170 with the periphery being welded air-tightly to define a periphery-welded portion 172, where the periphery of the observation window 178 is welded air-tightly to define a periphery-welded portion 179. (b) shows the bag-like pressing chamber 15 that is folded toward the back side at the line A-A' in (a). (c) is a horizontal cross section taken along the line B-B'.

The chamber 170 is provided with an inlet 175 to which an air-feeding tube 176 is connected. The chamber 170 is provided with a fixing portion 171 and a bag fixing portion 173 respectively on its right and left ends and stopper belts 181, 182, 183 on its lower end. The chamber 170 is folded toward the back side at the line A-A' and the bag fixing portion 173 is adhered to the fixing portion 171, where the stopper belts 181, 182, 183 are fixed to each other at positions facing each other to form fall-stops 185, 186.

(c) is a horizontal cross section of the bag-like pressing chamber 15 having the observation window 178 from which an infusion bag (not shown) set on the inner side thereof can be visually checked, so that a remaining amount of the infusion bag can be checked even though the pressing bag has a bag-like shape.

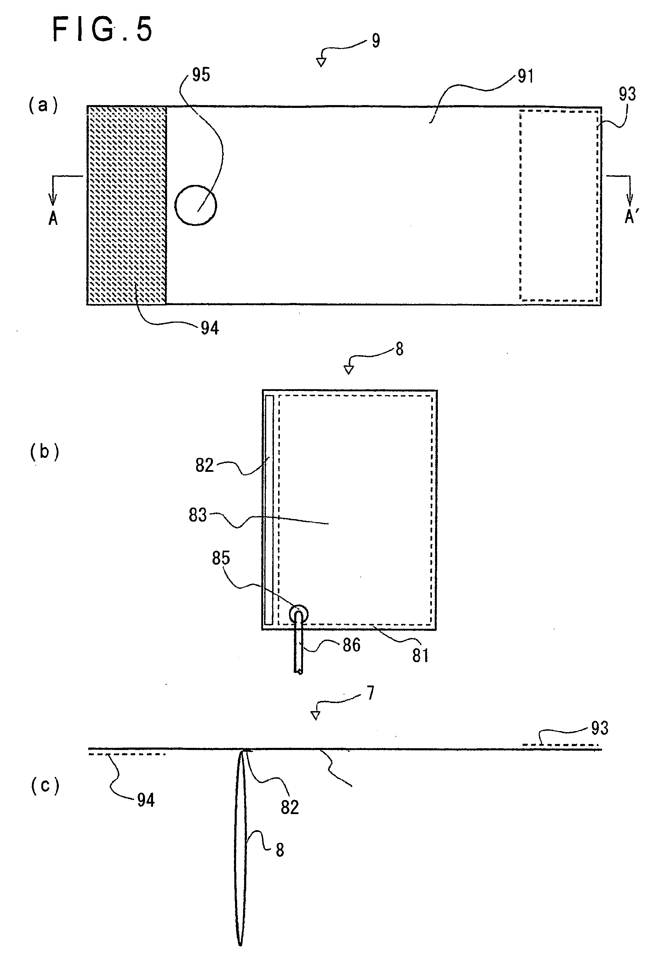

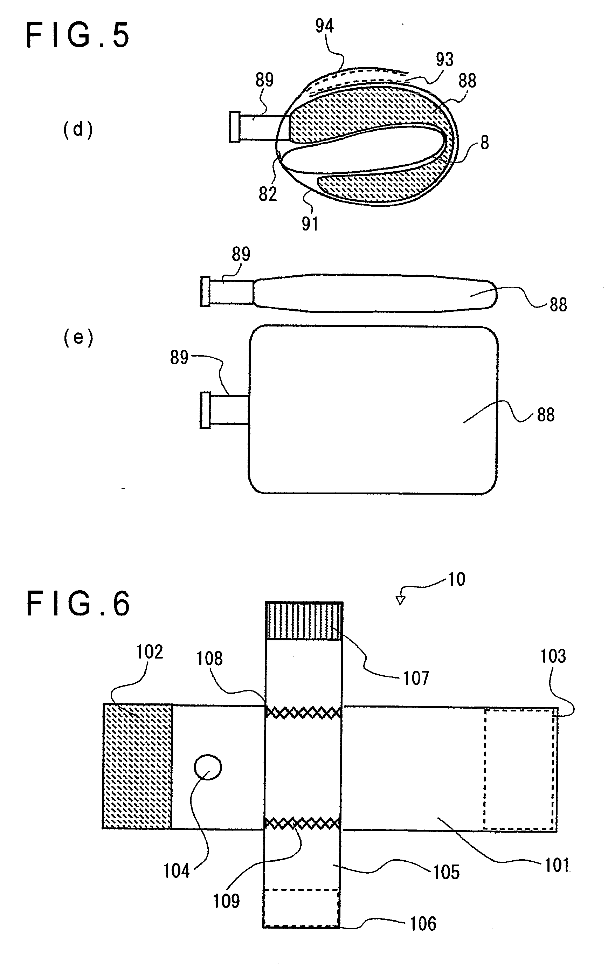

Fig. 5 shows a forth embodiment of a pressing bag of the present invention, which is a pressing bag 7 that is devised for a large-volume infusion bag. (a) is a front view showing an outer layer 9 that is provided with a hole 95 through which a neck portion 89 of a large-volume infusion bag 88 is inserted. (b) is a front view of a pressing chamber 8. (c) is a horizontal cross section of the pressing bag 7 which is seen from the line A-A' of the outer layer 9, in which a fixing portion 82 of the pressing chamber 8 is fixed to the outer layer 9. (d) is a horizontal cross section seen from the line A-A' of the outer layer 9 and showing an usage example of the pressing bag 7, in which the neck portion 89 of the infusion bag 88 is set in the hole 95 of the pressing bag 7 and the infusion bag 88 is folded in a manner holding the pressing chamber 8 on the inner side thereof, surrounded by the outer layer 9 and fastened with planar fasteners 93, 94, and then the pressing chamber 8 is inflated by feeding the air.

The outer layer 9 shown in (a) is provided with the planar fasteners 93, 94 on one end of a surface and the other end of the opposite surface of an outer layer belt 91 that has a longer side in the lateral direction. The outer layer 9 is also provided with the hole 95 through which the neck portion 89 of the infusion bag 88 is inserted. A position and the number of the hole may be determined depending on a type of the infusion bag.

The arrangement of the pressing chamber 8 shown in (b) is similar to the pressing chamber 2 shown in Fig. 1 (a), the size of which is determined depending on the size of the infusion bag 88 to be set. Since the infusion bag 88 is folded in the manner holding the pressing chamber 8 on the inner side thereof as shown in (d), the pressing bag can be compact. (e) shows a cross section of the infusion bag 88 which is seen in the lateral direction and a front view of the same.

Fig. 6 is a front view of an outer layer 10. An outer layer belt 101 has a longer side extending in the lateral direction and is provided with planar fasteners 102, 103 on both ends thereof so as to fasten to each other for surrounding. A restricting belt 105 is fixed to the outer layer belt 101 with fixing portions 108, 109 so that the outer layer belt 105 oriented in the axial direction. A hole 104 shown in the figure is formed in accordance with an infusion bag to be used.

The restricting belt 105 is provided with planar fasteners 102, 103 on both ends thereof for fastening when surrounding a pressing chamber (not shown) and an infusion bag (not shown). Although the restricting belt 105 is fixed to the outer layer belt 101, it may alternatively be arranged so that a part of the outer layer belt 101 is projected in the axial direction and the planar fasteners 106, 107 are provided to both ends of the projected part (not shown).

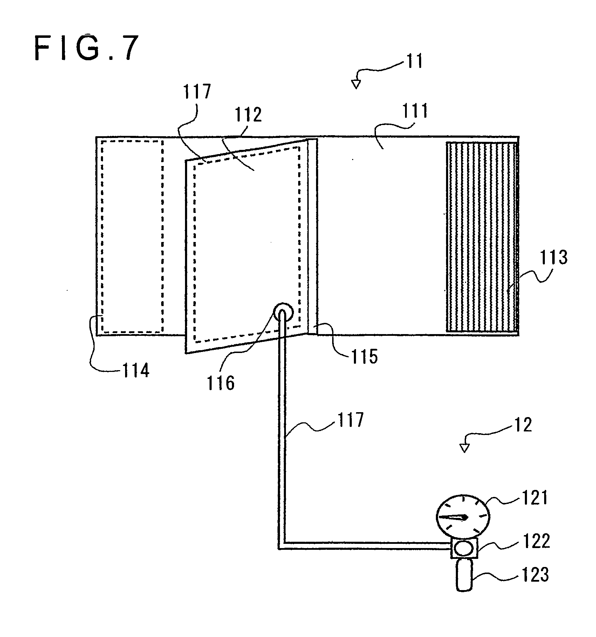

Now, a constant-pressure pressing bag system as another aspect of the invention will be described. Fig. 7 shows an example in which a gas feeder 12 is connected to the pressing bag 1 having the same arrangement in the first embodiment of the present invention, where planar fasteners 113, 114 are provided to both ends of the outer layer belt 111 so as to fasten to each other for surrounding, a pressing chamber 112 is formed air-tightly with the periphery being welded, and the pressing chamber 112 is provided air-tightly with an inlet 116 to which an air-feeding tube 117 is connected.

The gas feeder 12 includes a small gas cartridge 123 that is connected to a decompression valve 122 incorporating a pressure gauge 121. A pressurized gas to be fed from the gas cartridge 123 is controlled to a target pressure using the decompression valve 122 by monitoring the pressure gauge 121. The controlled pressurized gas is fed to the pressing chamber 112 of the pressing bag 11 through the connected air-feeding tube 117. If the decompression valve fixes the pressure to be constant, the pressure gauge becomes unnecessary.

The pressurized gas may be fed from an in-hospital piping, instead of from the gas cartridge 123, to the decompression valve 122 via a connecting tube (not shown) to be controlled to a target pressure, and then fed to the pressing chamber 112 of the pressing bag 11.

By constantly feeding the constant-pressure gas to the pressing bag 11 using the gas feeder 12, the constant-pressure pressing bag system capable of constantly pressing the infusion bag with the constant pressure can be provided.

In a conventional pressing bag, when infusion is sent out from the infusion bag, the internal pressure of the pressing chamber is lowered. With such arrangement, the conventional pressing bag requires a periodical check for the pressure of the pressing chamber and to perform a controlling operation for feeding the air using a manual rubber-bulb pump when the pressure is not enough. Especially, when the infusion rate is high, frequencies of the checking and the controlling operation for feeding the air using the manual rubber-bulb pump, which has burdened a health personnel with a large work load. In addition, conventional pressing bags using large-volume pressing chambers require large consumption amount of expensive gas, which has been unsuitable for the use of gas cartridges.

On the other hand, the constant-pressure pressing bag system using in combination a gas-saving and non-bulky outer-layer type pressing bag (as represented by the pressing bag 11) and the gas feeder 12 does not require the above-described controlling operation of the pressing bag.

### Industrial Applicability

Pressure bags are essentially used for infusion, blood-transfusion and the like in the medical field.

### Brief Description of Drawings

Fig. 1 is an illustration schematically showing an example of a pressing bag 1 according to the present invention;
Fig. 2 is an illustration schematically showing an example of a pressing bag for multiple infusion bags according to the present invention;
Fig. 3 is an illustration schematically showing an example of a pressing bag having a bag-like pressing chamber according to the present invention;
Fig. 4 is an illustration schematically showing an example of a pressing bag having a bag-like pressing chamber with an observation window according to the present invention;
Fig. 5 is an illustration schematically showing an example of a pressing bag for a large-volume infusion bag in preparation according to the present invention;
Fig. 6 is an illustration schematically showing an example of a pressing bag having an outer layer with a restricting belt according to the present invention; and
Fig. 7 is an illustration schematically showing an example of a constant-pressure pressing bag system according to the present invention.

### Explanation of Codes

- 1: pressing bag
- 2: pressing chamber
- 3: outer layer
- 4: pressing bag with two chambers
- 5: pressing chamber with two chambers
- 6: outer layer
- 7: pressing bag
- 8: pressing chamber
- 9: outer layer
- 10: outer layer with restricting belt
- 11: pressing bag
- 12: gas feeder
- 13: pressing bag having bag-like pressing chamber
- 14: bag-like pressing chamber
- 15: bag-like pressing chamber with observation window

## Claims

1. A pressing bag, comprising:
a pressing chamber adapted to be inflated by air-feeding; and
an outer layer that has a rectangular shape and includes fasteners on one end side of a front surface and the other end side of a back surface of shorter sides of the rectangular shape, the outer layer having such length as to surround the pressing chamber, wherein
a fixing portion of the pressing chamber is fixed in an axial direction to the outer layer, and
the outer layer surrounds the pressing chamber in such a manner that the pressing chamber contacts with an infusion bag, the outer layer being fastened by the fasteners.

2. A pressing bag, wherein two or three fixing portions of a pressing chamber that is adapted to be inflated by air-feeding are fixed to an outer layer, the fixing portions being fixed in parallel to each other in an axial direction.

3. A pressing bag, wherein
a pressing chamber is divided into two communicating chambers by a partition that is formed in an axial direction by welding slightly shortly so as not to reach a peripheral side of the pressing chamber, the partition formed substantially at the middle in a lateral direction of the pressing chamber, and
a fixing portion of the partition is fixed to an outer layer.

4. A pressing bag, wherein a fixing portion of a bag-like pressing chamber that includes one bag-like chamber adapted to be inflated by air-feeding is fixed to an outer layer in an axial direction.

5. A pressing bag, wherein
a bag-like pressing chamber is adapted to be inflated by air-feeding and divided into two communicating bag-like chambers by a partition that is formed in an axial direction by welding slightly shortly so as not to reach a peripheral side of the bag-like pressing chamber, the partition formed substantially at the middle in a lateral direction of the bag-like pressing chamber, and
a fixing portion of the partition of the bag-like pressing chamber is fixed to an outer layer.

6. The pressing bag according to claims 4 and 5, wherein
the bag-like chamber of the bag-like pressing chamber is adapted to be inflated by the air-feeding and provided with an observation window with a periphery thereof being welded, and
the fixing portion of the bag-like pressing chamber is fixed to the outer layer in the axial direction.

7. The pressing bag according to claims 1 to 3, wherein a part of a lower end of the pressing chamber that is adapted to be inflated by the air-feeding and a part of a lower end of the outer layer are adhered to define a fall stop.

8. The pressing bag according to claims 4 to 6, wherein parts of a lower end of the bag-like pressing chamber are adhered to define a fall stop.

9. A pressing bag, comprising:
a pressing chamber adapted to be inflated by air-feeding; and
an outer layer that has a rectangular shape and includes fasteners formed on one end side of a front surface and the other end side of a back surface of shorter sides of the rectangular shape, the one end side being provided with a hole, wherein
a fixing portion of the pressing chamber is fixed in an axial direction to the outer layer, and
an infusion bag is set so as to hold the pressing chamber on an inner side thereof, a neck portion of the infusion bag being inserted through and brought out of the hole, and the infusion bag is surrounded by the outer layer and fastened by the fasteners.

10. The pressing bag according to claim 9 wherein a part of the rectangular outer layer is formed lengthwise in the axial direction to form a restricting belt, the restricting belt adapted to be fastened with fasteners provided on one end side of a front surface and the other end side of a back surface of the restricting belt.

11. A pressing bag system, comprising a gas feeder for controlling a pressure of a gas and feeding the gas, the gas feeder connected to the pressing bag according to any one of claims 1 to 9 in order to inflate the pressing bag by air-feeding.
